# EUROPEAN PATENT APPLICATION

(11) **EP 3 985 098 A1**
(43) Date of publication of application: **20.04.2022**
(21) Application number: 20823576.2
(22) Date of filing: 15.06.2020
(51) Int. Cl.: C12M 1/42

(54) **METHOD AND DEVICE FOR CELL OR MICROVESICLE ISOLATION**

(30) Priority: 13.06.2019 CN 201910512145
(71) Applicant: Savelife Biotechnology Co., Limited, Hong Kong (HK)
(72) Inventor: DUAN, Xuexin, Tianjin 300072 (CN); YANG, Yang, Tianjin 300072 (CN)
(74) Representative: Laine IP Oy
(86) International application number: PCT/CN2020/096176
(87) International publication number: WO 2020/249130

(57) **Abstract**

Disclosed are a microfluidic system and method for isolating target cells or vesicles in a fluid. The system of the present invention comprises a fluid passageway having an inlet and an outlet; one or more ultra-high frequency acoustic resonator capable of generating bulk acoustic waves in the fluid passageway at a frequency of about 0.5-50 GHz; a power regulator which adjusts the power of the bulk acoustic waves generated by the ultra-high frequency resonator; and a flow rate regulating device that regulates the velocity of the solution flowing through the bulk acoustic wave region. Adjusting the power of the generated bulk acoustic waves by means of the power regulator and/or adjusting the velocity of the solution flowing through the bulk acoustic wave region by means of the flow rate regulating device allow cells or vesicles to stay in a bulk acoustic wave-affected region. The system and method of the present invention can capture and release cells or vesicles in a solution, and further process and analyze the obtained cells or vesicles.

## Description

This application claims priority to the following Chinese patent application: Application No. 201910512145.2, filed on June 13, 2019 with a title of "Method and device for cell or microvesicle isolation", the entire contents of which are incorporated by reference in this application.

### Technical Field

The present invention relates to the field of cell research methodology and medical devices. Specifically, the invention relates to a microfluidic system for the isolation and analysis of cells or microvesicles and a method for using said system to isolate and analyze cells or microvesicles.

### Background Technology

The manipulation of biological particles, particularly cells or microvesicles, is one of the most fundamental biotechnologies and is also a technology for cutting-edge research. There are a variety of methods to achieve manipulation of biological particles, such as cells or microvesicles, including electrical methods, acoustic methods, optical methods, magnetic methods, chemical methods, fluid manipulation methods, and others. For example, the manipulation by acoustic surface wave and dielectrophoresis is related to the radius of the particles, it is better for particles of micron size and not good for particles of nanometer size; electrophoresis and other methods only work when the particles are electrically charged, so it is less distinguishable for different substances that are not electrically charged or have similar charge. The distinction between different substances that are not charged or have similar charge is poor. Optical manipulation methods are quite accurate, but generate more heat during manipulation, which has a greater impact on the activity of biomolecules, and the optical system is more complex and difficult to integrate with microfluidic devices. Each method has limitations when used alone, so in most cases, the use of a combination of different methods will lead to better sample differentiation and manipulation.

Single-cell manipulation is an area that has received increasing attention in recent years. Manipulation of single cells or microvesicles is important for studies such as single cell analysis, drug development, organoids and intercellular interactions. Among these, cell-to-cell specificity-based studies (e.g., studies of cell morphology, surface adhesion, migration rate, protein expression and gene expression of individual cells) rely heavily on single-cell manipulation techniques. However, traditional methods of extracting single cells are cumbersome, time consuming, laborious and inefficient.

Acousto-fluidic techniques such as surface acoustic waves and acoustophoresis based on standing waves in fluids have been used for the separation, extraction and manipulation of microparticles and cells. However, these existing techniques use devices that are large and difficult to miniaturize.

Therefore, there is an urgent need for a system and method to enable the capture of cells or microvesicles, especially single cells or vesicles, to facilitate further processing and analysis of the captured single cells or vesicles.

### Summary of the Invention

The present invention is the first to discover that flexible particles such as cells in solution can be effectively manipulated and separated in a microfluidic system using ultra-high frequency bulk acoustic waves, thus providing methods and systems for separating and "capturing" target cells. The method and system of the present invention can also be used to precisely control the type and number of cells captured, and is particularly suitable for obtaining a single or limited number of cells.

Specifically, the present invention provides a method for isolating a target cell or vesicle, comprising.
(1) allowing a solution containing cells or vesicles to flow through a microfluidic device, said device comprising :
   a fluid channel having an inlet and an outlet;
   one or more ultra-high frequency bulk acoustic wave resonators provided on a wall of said fluid channel, said ultra-high frequency bulk acoustic wave resonators being capable of generating bulk acoustic waves in said fluid channel with a frequency of about 0.5-50 GHz and transmitted to the opposite side of said fluid channel;
(2) said UHF resonator emits a bulk acoustic wave transmitted to said wall on the opposite side of said fluid channel, creating a vortex in the solution in the area affected by the bulk acoustic wave;
(3) causing cells or vesicles to remain in the area affected by the bulk acoustic wave by adjusting the power of the bulk acoustic waves (e.g. by means of a power regulator) and/or adjusting the velocity of flow of said solution through the area affected by the bulk acoustic wave (e.g. by means of a flow rate regulating device).

In one aspect of the present invention, it is also included the step of "releasing" said flexible particles from the retaining position, i.e. "releasing" said flexible particles. For example, said captured flexible particles can be released and go into a desired channel or container.

The UHF bulk acoustic resonator of the present invention is a resonator capable of generating acoustic waves at frequencies above 0.5 GHz (preferably above 1 GHz), for example at frequencies of 0.5 to 50 GHz. Said UHF bulk acoustic wave resonator can be a thin-film bulk acoustic wave resonator or solid-state assembly type resonator.

Said microfluidic device typically includes a power adjusting device that regulates the power of the bulk acoustic wave generated by said UHF resonator.

Said microfluidic device typically includes a flow rate adjusting device that regulates the rate of flow of said solution through the region affected by the bulk acoustic wave.

Flexible particles are nano- or micron-sized particles with deformation properties. The flexible particles may be artificial or natural. Said particles may be micro-agglomerates with a membrane structure, in particular micro-agglomerates with a lipid bilayer or lipid-like bilayer. The flexible particles covered by the present invention typically have a diameter of about 0.01-30 um, preferably 0.5-25 um, more preferably 0.8-20 um. In one aspect of the present invention, said flexible particles are naturally occurring particles such as cells or cellular vesicles released by cells into the external environment, including exosomes, microvesicles, vesicles, membrane vesicles, prostatic vesicles, microparticles, intraluminal vesicles, intranuclear body-like vesicles, or cytosolic vesicles. These cell-associated vesicles are vesicle-like vesicles with a double-layer membrane structure that are shed from the cell membrane or secreted by the cell. The vesicles typically have a diameter of about 30-1000 nm, such as having a diameter of about 800-1000 nm.

In one aspect of the present invention, said cell comprises a cell cluster. Said cell cluster typically comprises several, e.g., 2, 3, 4, 5, 6, 7, 8, 9 or 10, cells. In one aspect of the present invention, said vesicles comprise a cluster of vesicles. Said cluster of vesicles typically comprises several, e.g. 2-50, vesicles.

In one aspect of the present invention, wherein said cells or vesicles have a diameter of about 0.8-30 um, for example 1-25 um, and for example 5-20 um.

In one aspect of the present invention, said power adjusting device outputs power about 0.5-800 mW, preferably 0.5-500 mW, more preferably 0.5-350 mW.

In one aspect of the present invention, wherein said flow rate adjusting device adjusts the speed of said solution flow through the bulk acoustic region to be about 0.1-10 mm/s, preferably about 0.3-5 mm/s, more preferably about 0.5-2.5 mm/s.

In one aspect of the present invention, wherein said flow rate adjusting device regulates the rate of flow of said solution through the bulk acoustic region to be about 0.1-100 µL/min, preferably about 0.1-50 µL/min, more preferably about 0.5-20 µL/min.

In one aspect of the present invention, wherein the height of said fluid channel is about 1.5-10 times the diameter of said cell or vesicle, preferably about 1.5-6 times the diameter, more preferably about 2-4 times the diameter.

In one aspect of the present invention, said fluid channel of the microfluidic device has a height of about 25-200µm, preferably about 25-100µm, more preferably about 30-80µm, for example about 40-60µm.

In one aspect of the present invention, said UHF bulk acoustic wave resonator has a bulk acoustic wave generation area of about 500-200000 µm², preferably about 5000-50000 µm², most preferably about 10000-25000 µm².

In one aspect of the present invention, said inlet comprises a sample inlet and auxiliary solution inlet provided on one or both sides of said sample inlet. The auxiliary solution may be a liquid such as a buffer, for example. The auxiliary solution can be used to resuspend the "captured" cells or vesicles or to add reagents for treating the "captured" cells or vesicles, such as fluorescent markers that specifically identify said cells or vesicles or their specific markers. The auxiliary solution can also be used to control the direction and extent of flow of the sample fluid in the microfluidic channel based on sheath flow phenomenom.

In one aspect of the present invention, the aforementioned method is used to isolate and obtain a single cell or several (e.g. 2-100, preferably 2-10) cells, or to isolate and obtain a single or several (e.g. 2-100, preferably 2-10) cell clusters.

In one aspect of the present invention, the foregoing method further comprises selectively capturing cells or vesicles of different species or of different nature (e.g. of different size or density, etc.) in said bulk acoustic affected region. In yet another aspect of the present invention, it further comprises controlling the number of cells or vesicles that remain in said bulk acoustic wave affected region. In yet another aspect of the present invention, the cells or vesicles and the number thereof that remain in said bulk acoustic wave affected region may be selected by one of the following means or any combination thereof:
(a) regulating the power of the bulk acoustic wave;
(b) regulating the time of the generation of the bulk acoustic waves;
(c) regulating the speed of flow of said solution through the region of the bulk sound waves.

In one aspect of the invention, the method described above controls the cells or vesicles that remain in the region affected by said bulk sound waves to be a single or several (e.g., 1, 2, 3, 4, 5, 6, 7, 8, 9, 10, or 11-20) cells. In yet another aspect thereof, the velocity of the regulated solution flow through the bulk acoustic wave region is about 0.1-10 mm/s, preferably about 0.5-3 mm/s. In yet another aspect thereof, the power of the bulk acoustic wave is about 0.1-500 mW, preferably about 0.5-200 mW. In yet another aspect thereof, the velocity of the solution flow through the bulk acoustic wave region is about 0.1-10 mm/s, preferably about 0.5-3 mm/s. preferably about 0.5-3 mm/s, and the power of the bulk acoustic wave is about 0.1-500 mW, preferably about 0.5-200 mW.

In one aspect of the present invention, the foregoing method further comprises causing the remaining cell or vesicle to leave the remaing position, i.e. releasing said cell or vesicle. In yet another aspect of the present invention, said cell or vesicle may be released by one or any combination thereof by:
(a) stopping the bulk acoustic wave;
(b) decreasing the power of said bulk sound waves;
(c) increasing the velocity of the solution flow through the region of the bulk acoustic wave.

In one aspect of the present invention, said method controllably releases remaining cells or vesicles, and in yet another aspect of the present invention, said method may control the type of cells or vesicles released, for example by releasing cells or vesicles of different sizes. In yet another aspect of the present invention, the "captured" cells are controlled to be released in order of size from small to large by adjusting the power of the bulk sound waves, for example, by decreasing the power of said bulk sound waves by a degree of about 5-20%. In a further aspect of the invention, said method may control the number of cells or vesicles released, for example by adjusting the power of the bulk sound waves and/or the flow rate of the solution, to control that the "captured" cells being released in different numbers, thereby controllably obtaining the desired number of cells or vesicles, in particular individual cells or vesicles.

In one aspect of the present invention, said method further comprises the step of dispersing the clusters of cells. The clusters of cells are dispersed, for example, by adjusting the power of the bulk acoustic waves generated by the UHF resonator.

In one aspect of the present invention, said method further comprises increasing the power of the bulk acoustic waves generated by the UHF resonator to disrupt the cell membrane or vesicle membrane to release intracellular or vesicle material, such as proteins or nucleic acids, etc.

In one aspect of the present invention, the solution in said method is a liquid containing the cells or vesicles to be captured, such as body fluid, whole blood, any blood grade containing cells, fragmented tumors, tumor cell suspensions, cell cultures, or culture supernatants. In yet another aspect of the present invention, said solution is blood, including whole blood or a blood fraction.

In one aspect of the present invention, said method is for separating cells or vesicles from a solution containing other particles or cells. In yet another aspect of the present invention, wherein said cells or vesicles to be isolated are in a ratio of less than 1:10⁴, preferably less than 1:10⁵, more preferably less than 1:10⁶, to said other particles or cells in the solution. In yet another aspect of the present invention, said cells or vesicles to be isolated are at a ratio of less than 10 cells/ml or less in the solution.

In one aspect of the present invention, when dealing with different particles or cells, the microfluidic device in said method is set up to capture the desired cells or vesicles at a different location (or referred to as the capture point) than the location other cells or vesicles leave the bulk acoustic action region (or referred to as the release point). Preferably, the location of other cells leaving the bulk acoustic wave action region is set upstream of the location where the desired cells are captured. In yet another aspect of the present invention, the bulk acoustic wave action region of the UHF bulk acoustic wave resonator of the microfluidic device in said method is set in such a way that the location where the desired cells are captured (or referred to as the capture point) is different from the location where the other cells leave the bulk acoustic wave action region (or referred to as the release point).

In the present invention, said cells include cells of plants or animals (e.g., mammals including humans), natural or cultured, as well as single-celled organisms such as bacteria, fungi, or simple multicellular organisms. In one aspect of the present invention, said cells are eukaryotic animal cells, preferably mammalian cells, more preferably human cells. In yet another aspect of the present invention, said cell is a blood cell, a stem cell, a somatic cell or a tumor cell, for example a circulating tumor cell (CTC). Said circulating tumor cell is selected from, but not limited to, the following epithelial cell cancers: prostate cancer, breast cancer, colon cancer, bladder cancer, ovarian cancer, kidney cancer, head and neck cancer, pancreatic cancer, and lung cancer. In yet another aspect of the present invention, said cells are placental trophoblast cells or nucleated red blood cells, and more preferably, said cells are derived from peripheral blood of a pregnant woman.

In one aspect of the present invention, said method further comprises using the isolated cells or vesicles for one or more of the following analyses: DNA and RNA amplification (e.g., rapid amplification of cDNA ends (RACE); parsimonious oligomeric primer PCR; mitochondrial DNA PCR; genomic PCR, digital PCR, RT-PCR, neighbor-joining PCR; immuno-PCR); sequencing. Immunochemistry; metabolite analysis; enzymatic analysis; reporter gene expression analysis; and hybridization studies.

In one aspect of the present invention, said method is used for the assembly of a cell or vesicle with a microcapsule, in particular a single cell or vesicle with a single microcapsule. In one aspect of the present invention, which comprises causing a single or several cells or vesicles to remain in the acoustically influenced region of a UHF resonator, and further comprising causing a single or several microcapsules to remain in the acoustically influenced region of the same UHF resonator, and then said single or several cells or vesicles forming a assembly with a single microcapsule, such as forming said single or several cells or vesicles at a capture point with a single microcapsules at the point of capture. Said method further comprises adjusting the parameters of the microfluidic device such that the assembled microcapsule-cell/vesicle is released.

Microcapsules are devices comprising a plurality of compartments (partitions) that are widely used for different molecular biology applications, including but not limited to: nucleic acid sequencing, protein sequencing, nucleic acid quantification, sequencing optimization, detection of gene expression, quantification of gene expression, and single cell analysis of genomic markers or expressed markers. Partitioning of microcapsules may comprise reagents containing one or more reagents (e.g., enzymes, unique identifiers such as barcodes, antibodies, etc.). The material for the microcapsules, particularly the material of the shell of the microcapsules, may enable the microcapsules to rupture in response to an applied stimulus. The reagents encapsulated in the microcapsules may be a variety of molecules, chemicals, particles, and elements suitable for sample preparation reactions of the analyte. For example, the microcapsules used in a sample preparation reaction for DNA sequencing of a target may contain one or more of the following reagents: enzymes, restriction enzymes, ligases, polymerases, fluorophores, oligonucleotide barcodes, buffers, etc. In another example, microcapsules used in sample preparation reactions for single cell analysis may contain: lysis buffer, decontaminants, fluorophores, oligonucleotide barcodes, ligases, proteases, heat-activatable proteases, protease or nuclease inhibitors, buffers, enzymes, antibodies, nanoparticles, etc. Microcapsules are typically encapsulated and prepared by emulsions or droplet-based microfluidics. Said emulsions or droplets comprise water-in-oil formulations or water-in-oil-in-water (W/O/W) double emulsion formulations, or said emulsions or droplets comprise polymers such as agarose or PEG. The microcapsules generally have a size from 10 nm to 100 µm. Preferably, in the method of the present invention, the microcapsules have a size from lµm to 100µm.

In one aspect of the present invention, the method described above comprises the steps of:
(a) causing a single or several cells or vesicles to remain in the region of action of the bulk acoustic wave of the UHF bulk acoustic wave resonator by adjusting the parameters, for example by adjusting the bulk acoustic wave power and/or flow rate.
(b) injecting gel microspheres from the sample inlet, adjusting the parameters, for example by adjusting the bulk acoustic wave power and flow rate, such that a single or several microcapsules remain in the bulk acoustic wave action region of the UHF bulk acoustic wave resonator, while preventing the cells or vesicles remaining in step (a) from being released.
(c) said microcapsules are assembled with said remaining cells or vesicles in the bulk acoustic wave action region of the UHF bulk acoustic wave resonator. The combination of said single or several cells or vesicles with a single microcapsule is typically formed at a capture point in the bulk acoustic wave action region of the resonator.

In one aspect of the present invention, the method described above further comprises the steps of:
(d) causing the combined microcapsule-cell/vesicle to be released by changing parameters of the microfluidic device, for example by reducing the bulk acoustic wave power or stopping the bulk acoustic wave.

The present invention also provides a kit comprising microcapsules and a UHF bulk acoustic wave resonator as previously defined. Said kit can be used to assemble microcapsules and cells or vesicles (particularly individual cells or vesicles), and used for one or more of the following analyses of cells or vesicles (particularly individual cells or vesicles): DNA and RNA amplification (e.g., rapid amplification of cDNA ends (RACE); simplex oligomeric primer PCR; mitochondrial DNA PCR; genomic PCR, digital PCR, RT -PCR, neighbor-joining PCR; immuno-PCR); sequencing; immunochemistry; metabolite analysis; enzymatic analysis; reporter gene expression analysis; and hybridization studies. Said kits may also contain reagents for said analyses.

The present invention also provides microfluidic devices for separating desired flexible particles from solution.

In one aspect of the present invention, provides a microfluidic device for isolating a target cell or vesicle, comprising:
a fluid channel having an inlet and an outlet;
one or more ultra-high frequency bulk acoustic wave resonators provided on a wall of said fluid channel, said ultra-high frequency bulk acoustic wave resonators being capable of generating bulk acoustic waves in said fluid channel with a frequency of about 0.5-50 GHz and transmitted to the opposite side of said fluid channel;
a power adjusting device which adjusts the power of said bulk acoustic waves generated by said UHF resonator;
a flow rate adjusting device which adjusts the velocity of said solution flowing through the region of the bulk acoustic wave,
said UHF resonator emitting bulk acoustic waves transmitted to the opposite wall of said fluid channel, causing the solution flowing through the bulk acoustic wave region to generate vortices, adjusting the power of the bulk acoustic waves by said adjusting device and/or adjusting the velocity of said solution flowing through the bulk acoustic wave region by said flow rate adjusting device, causing the cells or vesicles to stay in the bulk acoustic wave region.

The microfluidic device provided by the present invention is used to process biologically active cells or molecules and therefore has a setup or material for handling biologically active substances. For example, the inner surface of its flow channel may be made of biocompatible materials. Another example is that it has a design that prevents cross-contamination.

In one aspect of the present invention, said power adjusting device outputs power of about 0.5-800 mW, preferably 0.5-500 mW, more preferably 0.5-350 mW.

In one aspect of the present invention, said flow rate adjusting device adjusts the velocity of said solution flow through the bulk acoustic region to about 0.1-10 mm/s, preferably about 0.3-5 mm/s, more preferably about 0.5-2.5 mm/s.

In one aspect of the present invention, said flow rate adjustment device adjusts the rate of flow of said solution through the bulk acoustic region to about 0.1-100 µL/min, preferably about 0.1-50 µL/min, more preferably about 0.5-20 µL/min.

In one aspect of the present invention, wherein the height of said fluid channel is about 1.5-10 times the diameter of said cell or vesicle, preferably about 1.5-6 times the diameter, more preferably about 2-4 times the diameter.

In one aspect of the present invention, said fluid channel of said microfluidic device has a height of about 25-200µm, preferably about 25-100µm, more preferably about 30-80µm, for example about 40-60µm.

In one aspect of the present invention, said ultra-high frequency bulk acoustic wave resonator has a bulk acoustic wave generating area of about 500-200000 µm², preferably about 5000-50000 µm², most preferably about 10000-25000 µm².

In one aspect of the present invention, said inlet comprises a sample inlet and auxiliary solution inlet provided on one or both sides of said sample inlet.

In one aspect of the present invention, said microfluidic device is set in such a way that the location of the target cell or vesicle being captured is different from the location of other cells leaving the bulk acoustic wave action region.

In one aspect of the present invention, the location of other cells or vesicles leaving the bulk acoustic wave action region is set upstream of the location of the target cell or vesicle being captured.

In one aspect of the present invention, wherein said UHF bulk acoustic wave resonator is a thin film bulk acoustic wave resonator or a solid state assembly type resonator, such as a thickness stretching vibration mode acoustic wave resonator.

In one aspect of the present invention, wherein the thickness of the piezoelectric layer of the UHF bulk acoustic wave resonator of said apparatus ranges from 1 nm to 2 um.

### Reference sign of the drawings

100 Microfluidic device 101 Fluid channel 200 Chip housing 201 Coulter cell counter 202 UHF bulk acoustic wave resonator 203 Top electrode layer 204 Piezoelectric layer 205 Bottom electrode layer 206 Acoustic reflection layer Acoustic impedance layer 207 Bottom liner layer 208 Top 300 PCL controller 301 High frequency signal generator 302 Power amplifier 303 Impedance

meter 400 Liquid injection and flow rate regulation device 500 Acoustic jet 501 Vortex 600 Larger size particles 601 Medium size particles 602 Smaller size particles

### Brief Description of the Drawings

In order to more clearly illustrate the technical solutions in the embodiments or prior art of the present invention, the following is a brief description of the accompanying drawings for use in the description of the embodiments or prior art. It will be apparent that the accompanying drawings in the following description are some embodiments of the present invention, and that other drawings of the present invention are available to a person of ordinary skill in the art.

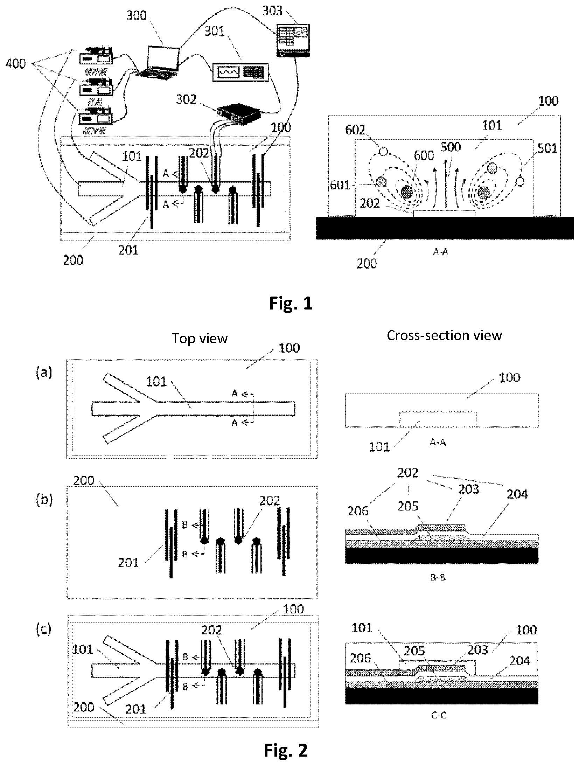

Figure 1 Schematic diagram of the structure of a microfluidic device system of an embodiment of the present application.
Figure 2 Schematic diagram of the structure of a UHF bulk acoustic wave resonator in a microfluidic device system of an embodiment of the present application; wherein, (a) represents a top view (left) and an A-A section (right) of the microfluidic channel of the microfluidic system shown in Figure 1; (b) represents a top view (left) of the UHF bulk acoustic wave resonator (wherein the black pentagonal area is the acoustic wave effecting region of the UHF bulk acoustic wave resonator) and B-B cross-sectional view (right); (c) top view (left) and cross-sectional view (right) of the microfluidic channel + UHF bulk acoustic resonator;

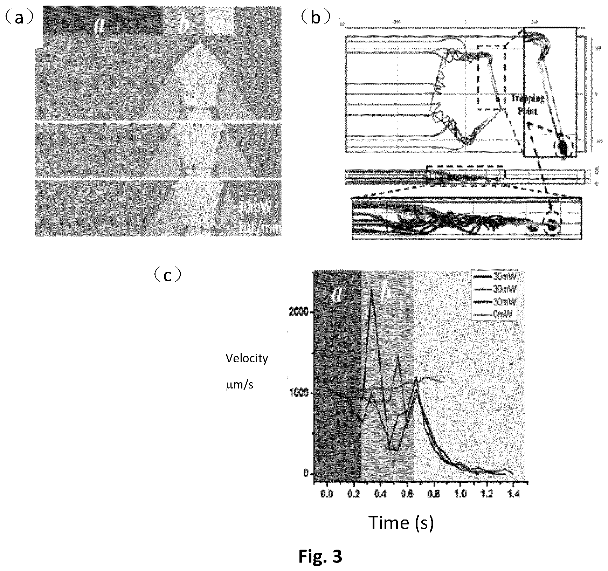

Figure 3 shows Hela cells entering the vortex channel in one of the microfluidic device systems of an embodiment of the present application. Figure 3(a) shows images of the motion trajectories of individual Hela cells in the vortex channel formed by the bulk acoustic waves generated by the UHF bulk acoustic wave resonator of the microfluidic device system (superimposed trajectories of individual cells at different times); Figure 3(b) shows a schematic diagram of the motion trajectories of Hela cells (superimposed trajectories of multiple cells) and an analysis graph; Figure 3(c) shows a time and velocity analysis graph of Hela cells.

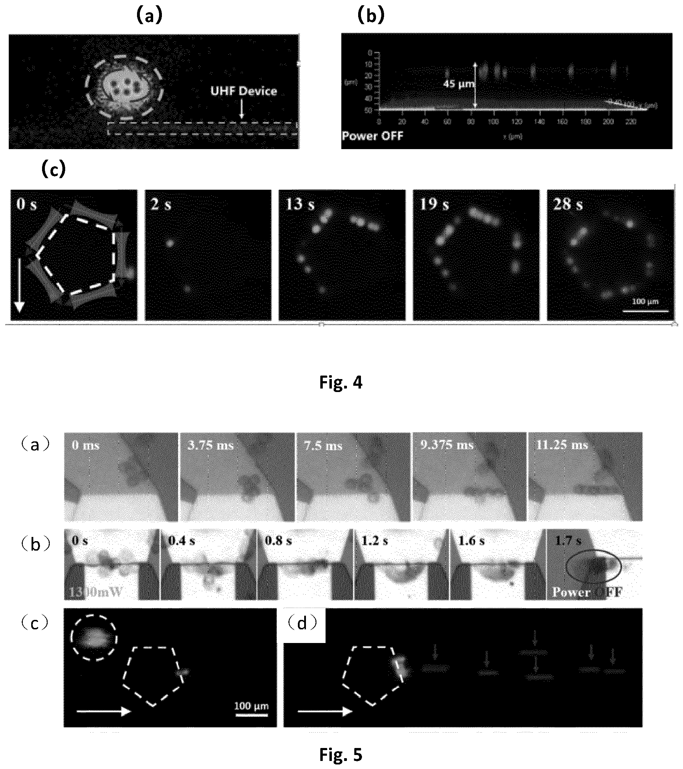

Figure 4 shows the movement of cells in a vortex channel in a microfluidic device system of an embodiment of the present application. Figure 4(a) shows images of cell movement in the vortex; Figure 4(b) shows cell settling after stopping the bulk acoustic wave; and Figure 4(c) shows the presence of multiple cells in the vortex tunnel.
Figure 5 shows controlled disintegration and release of cell clusters in a microfluidic device system of an embodiment of the present application. FIG. 5(a) shows that cell clusters are captured and disassembled into individual cells in the acoustic vortex tunnel under conditions of a bulk acoustic power of 66 mV. Figure 5(b) shows that the cells are disrupted by the vortex when the power of the bulk acoustic wave emitted from the UHF wave resonator is larger than a certain level. Figure 5(c) shows the fluorescence pattern of cell clusters captured in the acoustic vortex tunnel. FIG. 5(d) shows a fluorescence plot of cell clusters disintegrated into individual cells and the individual cells being released in the acoustic vortex tunneling (power 15 mV, flow rate 2 µL/min).

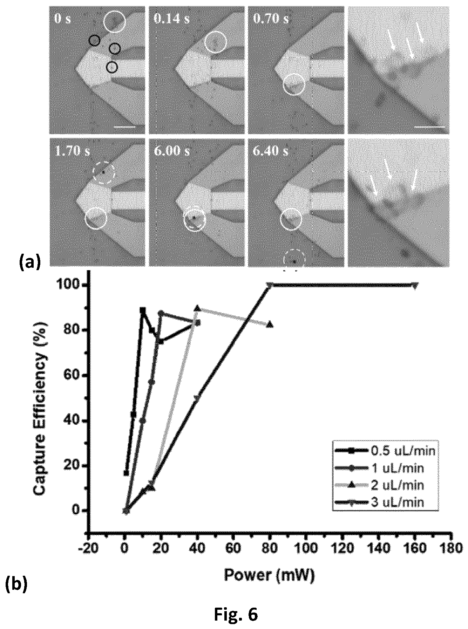

Figure 6 shows that a microfluidic device system provided by an embodiment of the present application is capable of isolating and capturing Hela cells from a blood mixture containing Hela cells and blood cells. Figure 6(a) shows that Hela cells are captured at the edge of the acoustic action region of the high frequency bulk acoustic resonator, while leukocytes and erythrocytes flow through the acoustic action region. Figure 6(b) shows a plot of the cell capture efficiency versus different bulk acoustic wave power and sample solution flow rate.

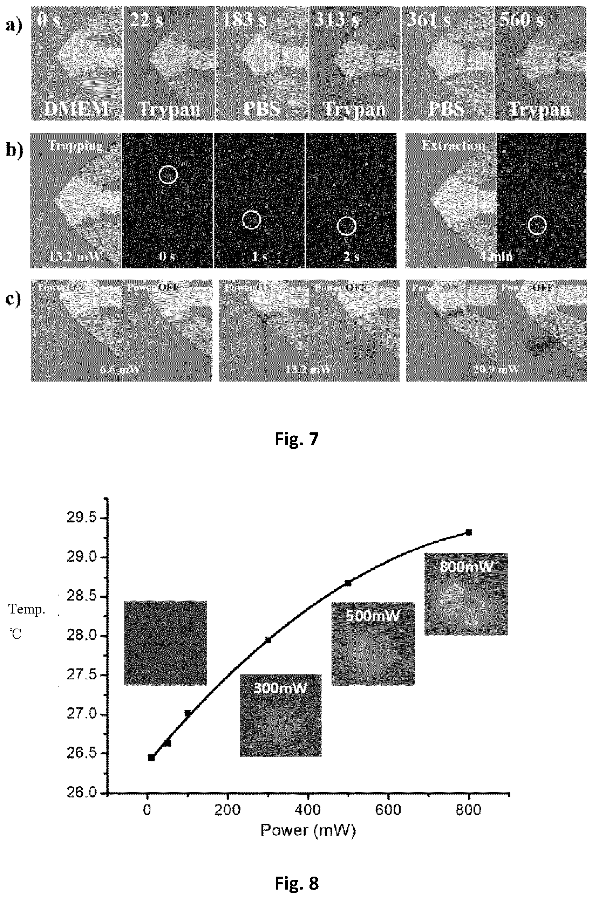

Figure 7 shows that a microfluidic device system of an embodiment of the present application is capable of separating and capturing different cells from the blood mixture. Figure 7(a) shows that Hela cells are trapped at the edge of the acoustic action region of the high frequency wave resonator and remain active at 560s. Figure 7(b) shows that the apparatus and method of the present invention can capture leukocytes and maintain activity. Figure 7(c) shows that the apparatus and method of the present invention can capture Hela cells, leukocytes and erythrocytes, respectively.
Figure 8 shows the temperature distribution around the UHF bulk acoustic resonator. Matlab software was used for image processing.

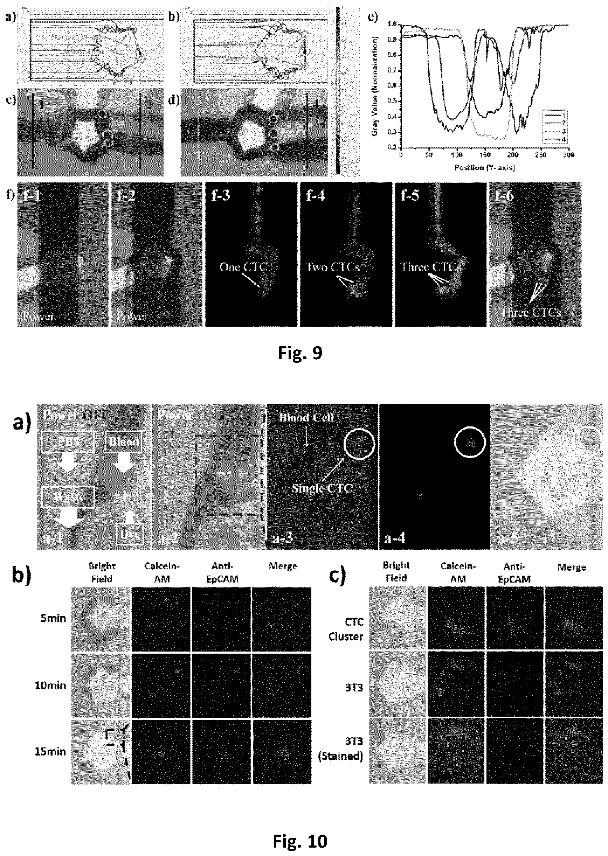

Figure 9 shows that a microfluidic device system of an embodiment of the present application is capable of isolating and capturing Hela cells from a mixture of whole blood. Figures 5(c) and 5(d) show photographs of Hela cells being captured and blood cells being released in two microfluidic channel setups, and Figures 5(a) and 5(b) are an analysis and schematic diagrams of Figures 5(c) and 5(d), respectively. Figure 5(e) shows the distribution of hemocytes upstream and downstream of the bulk acoustic wave action region (color line in Figures 5(c) and (d)).
Figure 10 shows that a microfluidic device system provided by an embodiment of this application is capable of capturing circulating tumor cells from blood samples of liver cancer patients. Figures 10 (a-1) and (a-2) show the solution in the flow channel when bulk acoustic waves and vortex flow are generated for the applied electrical power, respectively. Figure 10(a-3) shows captured individual cells stained green by Calcein-A. Figures 10(a-4) and 10(a-5) show fluorescent and bright field images, respectively. Figure 10(b) shows the staining of the captured cells. calcein-A stained green, while the red fluorescence of the anti-EpCAM marker represents the presence of the cancer marker EpCAM in the captured cells.

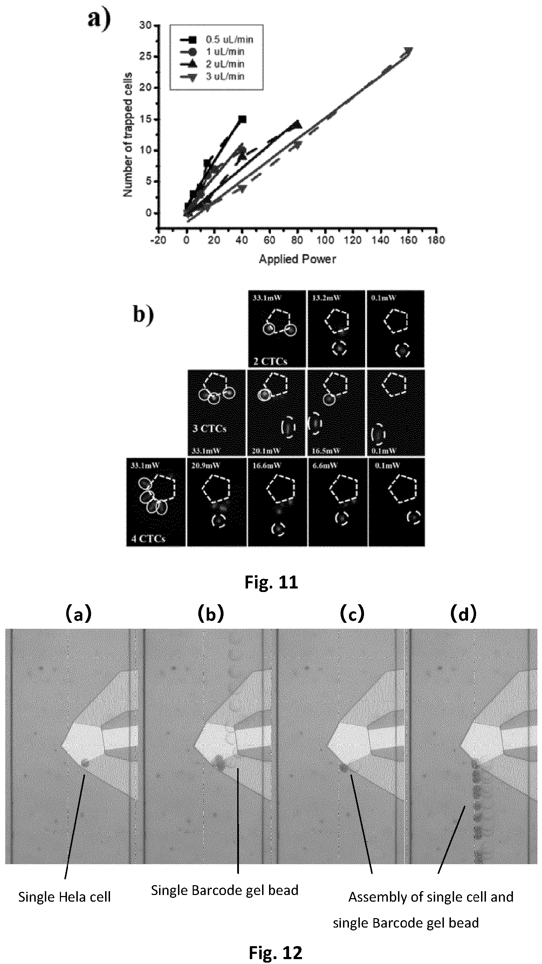

Figure 11 shows that the number of cells captured is linearly related to the power applied to the UHF bulk acoustic resonator under constant flow rate conditions.
FIG. 12 shows that a microfluidic device system provided by an embodiment of the present application assembles single or several cells with microcapsules in one-to-one mode.

### Detailed Description

The nature and benefits of the present disclosure are further described with reference to the following examples, which are intended to illustrate the invention provided herein and not to limit the scope of the present disclosure.

### Example 1 Experimental method and materials

Microfluidic channel and UHF bulk acoustic resonator preparation:
Microfluidic channels made of polydimethylsiloxane (PDMS) were prepared by soft lithography.

The bulk acoustic wave resonator devices are prepared by chemical vapor deposition, metal sputtering, and lithography on a silicon wafer. The specific methods are as follows.
1. The surface of the silicon wafer is thoroughly cleaned using a solution with a 3:1 volume ratio of concentrated sulfuric acid to hydrogen peroxide, which effectively removes organic and inorganic materials from the wafer.
2. On the cleaned silicon wafer, an aluminum nitride film is formed by surface sputtering, and then a silicon dioxide film is deposited using an ion-enhanced chemical vapor deposition method. Then, using the same method, the aluminum nitride film and the silicon dioxide film are deposited alternately to form a Bragg acoustic reflection structure with alternating layers of aluminum nitride and silicon dioxide.
3. On top of the Bragg reflector structure, a 600 nm molybdenum film is sputtered as the bottom electrode. Next, the molybdenum electrode film is photolithographed using standard photolithography techniques, including glue coating, exposure, and development, followed by etching to form a bottom electrode with a target pattern.
4. Another layer of aluminum nitride film is sputtered on the molybdenum electrode as a piezoelectric layer. The pattern is defined on the aluminum nitride film using dry etching.
5. The pattern on the mask plate is transferred using negative photoresist and then a 50 nm thick layer of titanium tungsten alloy is sputtered, which acts as an adhesion layer to increase the adhesion of the gold electrode. After that, a 300 nm thick layer of gold thin film of the upper electrode is formed by using vapor deposition. Finally, acetone is used to remove the gold film around the target pattern to form the gold electrode with the target pattern.

Finally, the bulk acoustic wave resonator device is integrated with the PDMS microchannel chip. The bulk acoustic wave resonator device is set in the middle of the channel.

The bulk acoustic wave resonator device is connected to a network analyzer using a standard SMA interface, and the frequency of the bulk acoustic wave emitted by the resonator device in the microchannel can be measured by testing the spectrum to find the resonance peak.

### Instruments and Materials

Signal generator: MXG Analog Signal Generator, Agilent, N5181A 100kHz-3GHz
Power amplifier: Mini-Circuits, with 35 dBm enhancement of the original RF source power
Syringe Pump: New Era Pump Systems, Inc., NE-1000

### Cells

Hela cell line: ATCC#CCL2, Guangzhou Genealogy Biotechnology Co.
3T3 cell line: ATCC#CRL-1658, Guangzhou Genealogy Biotechnology Co.
Cell culture: Cells were cultured in DMEM medium (Thermo) supplemented with 10% FBS (Thermo), 100 U/ml penicillin (Thermo) and 100ug/ml streptomycin (Thermo). The cell densities in culture ranged from 1x 10⁵/mL to 2x 10⁶/mL. For microfluidic experiments, dilution to 1×10⁵/mL was used for experiments. PBS buffer (Gibco).

### Cell or tumor markers or stains.

Calcein-AM (Beijing Solaibao Technology Co., Ltd., China)
Anti-EpCAM (Biolegend, USA)
4',6-diamidino-2-phenylindole (DAPI) (Invitrogen, USA)

### Example 2

In this embodiment of the present invention, a microfluidic device is provided which can be used to separate and capture flexible particles in solution, in particular flexible particles with a diameter of about 0.8-30 um. The flexible particles may be artificial or natural, and typically said particles are microclusters with a membrane structure, in particular microclusters with a lipid bilayer or a lipid-like bilayer. The flexible particles covered by the present invention typically have a diameter of about 0.8-30 um. In one aspect of the present invention, said flexible particles are naturally occurring particles, such as cells or vesicles released by cells into the extracellular environment.

The method and devices of the present invention can be used to separate and capture flexible particles in solution, for example to separate and capture target cells in blood. Another example is the separation and capture of vesicles in plasma.

As shown in Figure 1, said microfluidic device 100 includes a fluid channel 101, a UHF bulk acoustic wave resonator 202, a bulk acoustic wave power regulation device, and a liquid injection and flow rate adjusting device 400.

The microfluidic device provided by the present invention may be provided independently or may be part of a microfluidic system, for example in the form of a loadable chip. Microfluidic systems or devices can be used to contain and transport fluidic materials such as liquids with flow channel sizes in the micron or even nanometer range. Typical microfluidic systems and devices typically include structures and functional units of millimeter or smaller size.

The fluid channels, or microfluidic channels, of said microfluidic devices are generally closed except for openings for fluid entry and exit. The cross section of the fluid channel typically has dimensions of 0.1-500m, which can be of various shapes including elliptical, rectangular, square, triangular, circular, etc. Fluid channels can be prepared using a variety of known micropreparation techniques with materials including, but not limited to, silica, silicon, quartz, glass, or polymeric materials (e.g., PDMS, plastic, etc.). Said channels can be coated with a coating. The coating may modify the properties of the channel and may be patterned. For example, the coating may be hydrophilic, hydrophobic, magnetic, conductive, or biologically functionalized.

In one aspect of the present invention, said fluid channels of the microfluidic device have a height of about 20-200µm, preferably about 20-100µm, more preferably about 30-80µm, for example about 40-60µm. In one aspect, said microfluidic device is used to capture cells, said fluidic channel have a height of typically about 1.5-10 times the diameter of the target cell, preferably about 1.5-6 times the diameter, more preferably about 2-4 times the diameter.

In one aspect of the present invention, said fluid channel of the microfluidic device has a width of about 50-1000µm, preferably about 100-500µm, more preferably about 150-300µm.

The microfluidic channel 100 in this embodiment has an inlet and an outlet for solution entry and exit. Said inlet is connected to a fluid injection device for receiving the solution. Said inlet in this embodiment comprises a sample inlet 101 and a buffer inlet 102, wherein said buffer inlet is two inlets provided on either side of said sample inlet. Said microfluidic inlet setting facilitates passive focusing of the sample solution when passing through the sample inlet in the middle by this setting (sample flow in the middle and buffer flow on both sides).

As shown in Figure 1, the microfluidic device of this embodiment includes a liquid injection and flow rate adjustment device 400 for controlling the solution injection and controlling the flow rate of the solution. Said solution may be a solution containing a sample. For example, said sample is a solution containing cells to be captured. Said sample may comprise body fluids, whole blood, any blood fractions containing cells, tumor fragments, tumor cell suspensions, cell cultures or culture supernatants, etc. Said solution may be a variety of body fluids, including blood, tissue fluid, extracellular fluid, lymphatic fluid, cerebrospinal fluid, room water, urine, sweat, etc.

The flow rate of the injected solution can be controlled by means of an external pressure source, an internal pressure source, electrodynamics, or magnetic field dynamics. The external pressure source and internal pressure source can be a pump, such as a peristaltic pump, a syringe pump, or a pneumatic pump. In this embodiment, a syringe pump fine-tuned by a computer is used to control the flow rate of the liquid injection.

In the present invention, the flow rate of the solution ranges from about 0.1-10 mm/s, preferably from about 0.3-5 mm/s, more preferably from about 0.5-3 mm/s. In another aspect of the present invention, the flow rate of said solution ranges from about 0.1-100 µL/min, preferably from about 0.1-50 µL/min, more preferably from about 0.5-30 µL/min.

Said channels may be a single channel, or a plurality of channels arranged in parallel, wherein the outflow and inflow of solution and the flow rate thereof of each channel may be controlled jointly or independently as desired.

The microfluidic device of the present invention has one or more UHF bulk acoustic resonators 200, which are provided on one of the walls of the fluid channel (typically provided at the bottom of the flow channel). Said UHF bulk acoustic wave resonators may generate bulk acoustic waves at a frequency of about 0.5-50 GHz in said fluid channel which transmit to a wall on the opposite side of said fluid channel (typically the top of the flow channel).

The UHF bulk acoustic wave resonator that may be used in the present invention may be a thin film bulk acoustic wave resonator or a solid state assembly type resonator, such as a thickness stretching vibration mode acoustic wave resonator.

As shown in Figure 1, the microfluidic device of this embodiment has a plurality of UHF bulk acoustic wave resonators 202 disposed at the bottom of the flow channel.

Said UHF bulk acoustic wave resonators are bulk acoustic wave generating components that can generate bulk acoustic waves in said fluid channel that are transmitted to the opposite side of said fluid channel's wall.

As shown in the cross-section on the right side of Figure 2(b), said UHF bulk acoustic wave resonator includes an acoustic wave reflector layer 206, a bottom electrode layer 205, a piezoelectric layer 204 and a top electrode layer 203 provided sequentially from the bottom to the top. said bottom electrode layer, piezoelectric layer, top electrode layer and acoustic wave reflector layer overlap each other to form a bulk acoustic wave generation region. As shown in the top view on the left side of Figure 2(b), the top surface of said UHF bulk acoustic wave resonator is configured on the wall of the fluid channel to generate bulk acoustic waves emitted in a direction perpendicular to said wall to the opposite wall; in general, the area constituted by the top surface of the UHF bulk acoustic wave resonator is the bulk acoustic wave generation region, also referred to as the bulk acoustic wave region or the bulk acoustic wave effecting region in this specification. In one aspect of the present invention, said bulk acoustic wave action region has an area of about 500-200,000 µm², preferably about 5,000-50,000 µm², and most preferably about 10,000-25,000 µm². The bulk acoustic wave action region of this embodiment, as shown in Figure 2, is pentagonal in shape with sides of about 120 micrometers.

In the present invention, said shape of the bulk acoustic wave action region includes at least, but is not limited to, one of the following: a circle, an ellipse, a semicircle, a parabola, a polygon with an acute or obtuse angle at the vertex, a polygon with the vertex replaced by a circular arc, a polygon with any combination of an acute angle, a semicircle or a parabola at the vertex, or a repeatedly arranged square or circular array of the same shape. The present application provides the acoustic action area of the above-mentioned shapes, but other acoustic action areas of any shape are also within the scope of protection of the present application.

As shown in the right-hand section of Figure 2, the fluid channels of this embodiment have a plurality of UHF bulk acoustic wave resonators. In one aspect of the present invention, they are arranged in a straight line in the same direction as the direction of solution flow.

The UHF bulk acoustic wave resonator employed in this embodiment of the present invention is a thickness-stretching vibration mode in which a thin film layer of piezoelectric material is made by growing in the vertical direction and is excited by coupling the vertical electric field through the d33 piezoelectric coefficient. The UHF bulk acoustic resonator employed in the present invention can generate localized acoustic flow at the interface between the device and the liquid without a coupling medium or a coupling structure.

The UHF bulk acoustic wave resonator employed in the present invention produces a UHF bulk acoustic wave in the solution that is essentially free of standing waves. As shown in the right panel of Figure 1, the UHF resonator emits a bulk acoustic wave transmitted to the opposite wall of said fluid channel (e.g., the top of the flow channel), and the volume force generated by the acoustic wave attenuates in the fluid and causes an acoustic jet 500 to appear in the flow-through solution, resulting in a localized three-dimensional vortex 501 of the fluid in the microfluidic channel, and the successive vortex caused by the UHF bulk acoustic wave forms an acoustic fluid vortex channel. Since the vortex is generated by the volume force triggered by the acoustic wave attenuation, the central axis of the vortex is above the boundary of the bulk acoustic wave action, so the shape of the vortex channel is basically the same as the shape of the bulk acoustic wave effecting region, located above the boundary of the bulk acoustic wave action region. The acoustic fluid vortex is caused by the nonlinearity of the acoustic wave propagation in the liquid medium. And the strength of the amplitude of the acoustic wave directly determines the strength of the acoustic fluid vortex. By adjusting the applied power, the amplitude of the acoustic wave can be regulated, which in turn controls the speed of the acoustic fluid vortex. The particles in the vortex (including larger size particles 600, medium size particles 601, smaller size particles 602) are subjected to forces including the vortex generated fluid drag force (Stokes drag force), flow layer generated inertial drag force (inertial lift force) and acoustic radiation force caused by acoustic attenuation. When the forces on the particles in the fluid reach a certain equilibrium, the particles stop at the relevant position in the vortex channel and do not produce motion relative to the flow channel. The magnitude of the fluid drag force is positively related to the particle such as particle diameter, while the magnitude of the acoustic radiation force is positively related to the square of the particle size. As the size of the particle increases, the main-effecting force will change from fluid drag to acoustic radiation force, which pushes the particle to the center of the vortex. Larger particles are subjected to greater acoustic radiation force and thus move to the center of the vortex; while smaller particles rotate at the periphery under the action of the vortex drag force. Particles move along and downstream of the bulk acoustic effecting region under the action of the lateral drag force generated by the flow layer.

The fluid drag force and acoustic radiation force induced by the acoustic waves in the vortex channel can be deduced from certain formulae, but the inertial lift force generated by the acoustic fluid vortex (inertial lift force) can hardly be calculated, especially in the case of fluids containing complex compositions. Compared to the two-dimensional particle capture in the prior art, the method and apparatus of the present invention involve deformable cells or vesicles whose forces and trajectories in the acoustic fluid vortex and the channels formed thereof are more complex, due to the interaction between the individual vortices and the migration of particles between the vortices have an impact on capture of the particles in the fluid, especially in the presence of a large number of said cells or vesicles in the fluid: the cells or vesicles interact and influence each other due to collisions and other phenomena, and the interacting forces and motions in the vortex differ from those calculated and simulated theoretically, and the motion patterns and trajectories in the vortex channel are even more impossible to predict based on theoretical calculations and simulations.

The applicant unexpectedly found through experiments that, in the method and device of the present invention, when cells or vesicles in solution passing through the region of the acoustic fluid vortex channel caused by ultra-high frequency bulk acoustic waves, under the suitable conditions of flow velocity and bulk acoustic wave power, said cells or vesicles do not produce motion relative to the flow channel after reaching a steady state at a certain position(s) in the vortex channel, i.e., they are captured (the position where the cell or vesicle reaches a steady state can be called capture point). Other cells or vesicles in the solution that do not meet the steady state (e.g., cells or vesicles of smaller size) are released when they do not enter the vortex channel or leave the vortex channel at a position(s) in the vortex channel and move downstream under the lateral drag force generated by the laminar flow; the position where the cell or vesicle leaves the vortex channel is also called the release point. In the vortex channel, due to laminar flow, the cell or vesicle in the center of the vortex usually moves to the region of the vortex channel downstream of the fluid channel, i.e., the capture point is usually in the region of the vortex channel downstream of the fluid channel.

The inventors also discovered, unexpectedly, that the location where the cell or vesicle leaves the vortex channel is usually at a position where a jump point of the acoustic radiation force occurs. When the conditions such as vortex-generating bulk acoustic wave power, solution flow rate, fluid channel shape and size are fixed, the location where the cell or vesicle leaves the vortex channel is usually at a position where the vortex channel has a turn or corner. Without being bound by the theory, the applicant believes that the reason for this phenomenon is that at the turn or corner of the vortex channel, the direction of the vortex and/or the direction of acoustic radiation force suddenly change, and the larger cells or particles, which are already focused on the vortex center and subject to greater acoustic radiation force, are able to change their direction of motion with the vortex channel and quickly refocus to the center of the vortex channel after the turn; while the smaller particles or cells are more affected by the change in drag direction and easier to leave the vortex channel.

In one of the preferred embodiments of the present invention, said microfluidic device is set so that when dealing with cells or vesicles containing of different sizes, the location at which the target cell or vesicle is captured (capture point) is different from the location at which the non-target cell or vesicle leaves the bulk acoustic wave action region (release point).

In one of the preferred embodiments of the present invention, the bulk acoustic wave action region of the UHF bulk acoustic wave resonator of said microfluidic device is set in such a way that when dealing with cells or vesicles containing of different sizes, the location where the target cell or vesicle is captured (capture point) is different from the release point. For example, in the case of a UHF bulk acoustic wave resonator with a pentagonal bulk acoustic wave action region, one of the corners is placed symmetrically along the midline of the flow channel in the direction of the channel fluid inflow: the location of the non-target cells or vesicles leaving the bulk acoustic wave action region, or release point, is at the ends of the downstream edge along the fluid direction; while the target cells or vesicles are captured at the capture point in the middle of the downstream edge along the fluid direction. Capture point and release point do not overlap with each other. As a result, the non-target cells have minimal effect on the stability of the captured cells. In yet another preferred embodiment of the present invention, the capture point is set upstream of release point.

The way of setting the microfluidic device so said capture and release points are separated can be obtained by extrapolation from theoretical calculations or by means of actual experiments.

In the case of selectively isolating circulating tumor cells from blood, due to the high cell density in blood, if the release site coincides with the capture site, all cells will pass through the capture site, which will lead to strong intercellular interactions and the possibility of shedding of circulating tumor cells at the capture site due to the collision of a large number of erythrocytes and leukocytes. In one embodiment of the present invention, by separating the capture site from the release site, for example by setting the release site before the capture site, the non-targeted erythrocytes and leukocytes are carried away by the laminar flow without passing through the capture site, and only the target circulating tumor cells can reach the capture site.

In this way, the present applicant's inventors have discovered and provided methods to more efficiently separate target cells or vesicles in a sample, especially to separate a very small number or a very low percentage of target cells or vesicles from a sample containing other cells or vesicles.

In the present invention, the frequency of the thin film bulk acoustic wave resonator is mainly determined by the thickness and material of the piezoelectric layer. The thickness of the piezoelectric layer of the thin film bulk acoustic resonator used in the present invention ranges from 1 nm to 2 um. The frequency of the UHF bulk acoustic resonator of the present invention is in the range of about 0.5-50 GHz, preferably greater than 1 GHz - about 10 GHz.

The bulk acoustic wave generated by said UHF bulk acoustic wave resonator is driven by a signal from a high frequency signal generator. The pulsed voltage signal driving the resonator can be driven with pulse width modulation, which can produce any desired waveform, such as a sine wave, square wave, sawtooth wave, or triangle wave. The pulsed voltage signal can also have an amplitude modulation or frequency modulation start/stop capability to start or eliminate bulk acoustic waves.

The microfluidic device of the present invention further comprises a power regulation device which regulates the power of the bulk acoustic waves generated by said UHF resonator. In this embodiment, said power regulating device is a power amplifier having a power regulation function. In one aspect of the present invention, said power adjusting device has an output power of about 0.5-800 mW, preferably 0.5-500 mW, more preferably 0.5-300 mW. Due to the high energy conversion efficiency of the thin film bulk acoustic wave resonator which essentially cause no energy loss, the output power of said power adjusting device can be considered essentially as the output power of the thin film bulk acoustic wave resonator generating bulk acoustic waves in fluid. In the microfluidic device of the present invention, said power adjusting device may be connected to a high frequency signal generator. The output circuit of said power amplifier is connected to the bottom electrode, piezoelectric layer, and top electrode of said ultra-high frequency bulk acoustic wave resonator, respectively.

The microfluidic devices of the present invention may also include detection devices for detecting signals of characteristics of cells or markers carried by them in the sample. These characteristics may include molecular size, molecular weight, molecular magnetic moment, refractive index, conductivity, charge, absorbance, fluorescence, polarity, and other physical properties. For example, detection devices include detecting electrical detection devices, such as Coulter counters, for cell counting. The detection apparatus may also be a photodetector, which includes an illumination source and optical detection components for detecting physical parameters such as charge, absorbance, fluorescence, polarity, etc.

In the microfluidic device of the present invention as shown in Figures 1 and 2, there is a Coulter counter (whose device base is impedance meter 303) set at a specified distance within said microfluidic channel from the intersection of said sample inlet and said buffer inlet, and at a specified distance within said microfluidic channel from the outlet of said microfluidic channel. Coulter counters are sensors that use the electrical properties of cells different from the culture medium (or buffer) to achieve cell counting and detection. Structurally, the Coulter counter consists of multiple electrode strips, mostly two or three electrodes, and works on the principle that when cells pass through the electrodes they will displace the same volume of electrolyte, resulting in a change in medium impedance between the electrodes, which will generate a transient potential pulse that can be detected by an external impedance meter. Thus, cell counting can be achieved. Also, since the processing is compatible with the processing of bulk acoustic wave devices (UHF bulk acoustic wave resonators), it can be integrated on the same substrate to achieve the detection of cells and vesicles, etc.

### Example 3 Isolation of Cells in buffer

Hela cells were dissolved in DMEM culture medium and prepared as sample solution (cell concentration of ∼1^{∗}10⁵/mL). Hela cells were labeled with Calcein-AM in order to be observed of their movement. The samples were injected from the sample inlet into the microfluidic channel.

The height of the said microfluidic channel was 50 um. The UHF bulk acoustic wave resonator frequency was 1.83 GHz and the output power was 30 mW. The sample input flow rate was controlled to be 1 uL/min and approximately 0.67 mm/s.

The results are shown in Figure 3. Figure 3(a) shows the motion trajectory of a single Hela cell in the vortex channel formed by the bulk acoustic wave generated by the UHF wave resonator of the microfluidic device system. It is a superposition of multiple images of the same single cell at different times to show its movement trajectory. Figure 3(b) shows the schematic diagrams of the movement trajectories of different individual cells: the top view is shown in the upper panel and the side view in the lower panel. Figure 3(c) shows the time and velocity analysis of Hela cells.

In the microfluidic device of the present invention, the bulk acoustic waves generated by the UHF bulk acoustic wave resonator cause the solution to generate vortices. Each vortex connects to an adjacent vortex, forming an acoustic fluid vortex channel or vortex tunnel along the boundary of the acoustic wave effecting region of the UHF bulk acoustic wave resonator. Cells enter the vortex tunnel as they pass through the vortex acoustic action region and are captured at a location(s) in the vortex channel that does not produce motion relative to the flow channel under suitable flow velocity and bulk acoustic wave power conditions (the location where the cell or vesicle reaches a steady state can be referred to as the capture site or capture point). Cells in this capture position are captured because they are subjected to very weak forces near the lateral flow due to the interaction between the vortex and the laminar flow, and therefore no more forward (horizontal) movement occurs. Moreover, the captured cells are floating above the bottom of the microfluidic channel and are not in contact with the microfluidic channel.

The image sequence in Figure 3(a) demonstrates the moving trajectory of a single HeLa cell. The movement process can be divided into three phases occurring in regions a-b-c, as shown in Figure 3(a) and Figure 3(c). In region a, the cells move at a uniform velocity in the lateral flow. In region b, the direction of cell movement is changed by the vortexes. The cells enter the vortex tunnel generated by the acoustic waves and move along the vortex tunnel while focused on the 3-D axis of the tunnel. Cells with different initial positions have varying velocities as they enter the vortex array. Due to the difference in orientation relative to the vortex, cells with initial positions in the lower layers of the microchannel accelerate, while cells with initial positions in the upper layers slow down. The speed of cell movement gradually slows down in region c. Due to the tuning of the cell trajectory in the vortex tunnel, the process of cell deceleration and movement has good stability, and the cells with different entry initial positions have good consistency of deceleration and movement.

Figure 3(b) schematic diagram further illustrates the movement of cells in the flow channel under the action of bulk acoustic waves. Figure 3(b) is a superimposed schematic of the motion trajectories of different individual cells. The top view of Figure 3(b) shows the top view and the bottom view shows the side view. From the top view perspective, it can be seen that the trajectories of the cells are distributed only at the boundary of the acoustic wave effecting region of the UHF bulk acoustic resonator. From the side view perspective, it can be seen that the cells all float above the bottom of the channel and have no contact with the bottom.

### Example 4 Cell suspension and settling in a microfluidic channel

In prior art acoustic vortex methods, the motion of particles or cells in the z-axis of the microfluidic channel (i.e., the vertical direction of the flow channel) is ignored because in-plane acoustic flow is the dominant flow. However, the trajectory of the z-axis is important for single-cell operation and high-precision detection. Due to the unique TE vibration mode of the UHF bulk acoustic wave employed in the present invention, the dominant flow in the microfluidic system of the present invention is out-of-plane (out-of-plane). To better investigate the vortex tunneling generated by UHF bulk acoustic waves in the microfluidic system of the present invention, Calcein-AM-labeled Hela cells were used to observe and measure the cell motion in the z-axis using a confocal microscope (Leica, Germany). To capture the particle trajectories within the vortex tunneling, the x-z-t mode was used. In this mode, the capture speed is 37 frames per second.

The same experimental setup is similar with that in Example 3, except that the input flow rate of the sample containing Hela cells is reduced to 0.1 L/min. After the sample containing cells and the buffer were input into the microfluidic channel, the signal generator was turned on and the cells were captured in the acoustic action region. Then the pressures in inlet and outlet are adjusted to the same and the fluid is stagnant. The motion of the cells in the vortex is observed. Then the signal generator is turned off and the motion of the cells is observed.

The results are shown in Figure 4. Figure 4(a) shows the images of cell movement in the vortex, which is a composite stacked image (6 images, 27ms apart), where the red dots show the center of the particles in each frame, the green dashed circles indicate the range particle motion and the red arrows indicate the direction of particle motion. When the signal generator power is turned on, bulk acoustic waves are generated and form vortices and vortex tunnels within the flow channel, and the particles are relatively "stationary" suspended at a distance from the chip surface. As shown in Figure 4(b), when the power is turned off, the bulk acoustic wave disappears and the particle settles to the chip surface under the combined effect of gravity and buoyancy. The results show that the particles are indeed trapped in the vortex tunnel.

Figure 4(c) shows that the Calcein-AM-labeled Hela cells are trapped in the vortex tunnel. The tunnel has the same shape as the boundary of the acoustic wave generating region of the UHF bulk acoustic wave resonator.

For cell manipulation, cells in contact with the device may be killed by the negative effects triggered by the device, thus making it less biocompatible. In addition, cell-cell adhesion and cell-device surface adhesion caused by contact is a serious problem, especially for single-cell operations. The present invention provides methods that avoid the problems of cell-cell or cell-device surface contact and adhesion that occur in cell capture and separation operations.

### Example 5 Break of cells

As shown in Fig. 5(b), the inventors found in their experiments that when the power of the said UHF bulk acoustic wave resonator within the microfluidic system was increased to more than 800mW, for example, at 1300 mW, the cell membrane of the cell would be disrupted under the combined effect of the shear force of the high-speed vortex and the volume force generated by the velocity gradient, and the cell would be lysed and the intracellular substances such as proteins and nucleic acids would be released, which enters the downstream of the system with the fluid flow. The power causing the cell or vesicle to be disrupted is related to the volume of the cell or vesicle. Since cell fragmentation is mainly affected by the compression of acoustic radiation force, which is generally proportional to the cube of the cell or vesicle volume, the power of the UHF bulk acoustic resonator is higher for the destruction and fragmentation of cells or vesicles with smaller volumes.

In cellular research, in order to obtain information about the cell, such as DNA, RNA, and proteins, the cell disrupts the cell membrane, including the cell membrane and the nuclear membrane, allowing the material encapsulated within the membrane to be released for downstream analysis. For example, in DNA sequencing, it is necessary to disrupt the cell membrane by lysate to obtain DNA before participating in polymerase chain reaction; protein blotting, which is commonly used in protein assays, also requires obtaining proteins from the cells. Currently, chemical lysis methods are commonly used to lyse cells by adding different lysis solutions to achieve cell lysis. Compared to physical lysis methods, chemical methods have the potential to affect downstream analysis due to the introduction of exogenous agents. Among the physical methods, there are ultrasonic lysis, thermal lysis, and laser lysis, etc. The only method that can achieve accurate lysis of a small number of cells is laser lysis at present, which is costly and less compatible with microfluidic chips due to laser and optical path limitations.

Using the method and system of the present invention, it is possible to achieve precise lysis of single-cell and obtain information on the unique properties of individual cells.

### Example 6 Separation of different cells in diluted blood samples

The forces affecting the motion of cells in the acoustic vortex of the present invention, including the fluid drag force (Stokes drag force), the inertial drag force (inertial lift force) generated by the acoustic fluid vortex and the acoustic radiation force (acoustic radiation force) caused by the acoustic waves, are closely related to the size of the cells. It needs to be investigated whether the method of the present invention can be used to separate cells of different sizes in solution, especially in blood, and the precision of the separation.

Hela cells were dissolved in DMEM culture solution and mixed with 200-fold diluted whole blood to prepare test samples (adjusted for Hela cell content of about 1×10⁵/mL.). Hela cells were labeled with Calcein-AM (green). The blood was stained with DAPI and the leukocytes appeared blue under the viewing microscope.

Samples were injected into the microfluidic channel from the sample inlet. PBS buffer were injected into the microfluidic channel from the buffer inlet on both sides of the sample inlet. The injected sample is passively focused, which allows better effect of causing the sample flowing through the acoustic action region of the high-frequency bulk acoustic resonator.

Figure 6 shows Hela cells in a blood mixture containing Hela cells and blood cells are selectively captured by the apparatus and method of the present invention.

Specifically, FIG. 6(a) shows that Hela cells (indicated by solid circles in the figure) stay in the acoustic action region of the resonator, while leukocytes (indicated by dashed circles in the figure) and red blood cells continue to move in the direction of fluid. The rightmost plot in Figure 6(a) shows an enlargement of its left side plot. As can be seen in the figure, the 3 Hela cells (indicated by arrows) were captured very stably even though other cells in the blood (leukocytes and erythrocytes) try to enter and occupy the position.

In addition, as shown in Figure 6(b), the cell capture efficiency was correlated with different bulk acoustic power and sample solution flow rate. In this experiment, the capture efficiency was defined as the number of cells captured in the acoustic wave action region of a single UHF bulk acoustic wave resonator over an input of 10 target cells. Within a certain range, the cell capture efficiency increases with the applied power and decreases with the increase of the flow rate. The reason for this is that an increase in transverse flow rate triggers stronger shear forces, which can lead to cell escape. The increased power produces higher velocity vortices that counteract the negative effect of transverse flow on the vortex tunneling of the bulk acoustic waves. To obtain a stable capture region with high resolution, the applied power and the liquid lateral flow rate can be balanced.

With the microfluidic system and method of the present invention, target cells can be extracted from a mixed sample (e.g., blood) into another solution (e.g., buffer or fluorescent dye). Specifically, as shown in Figure 7(a), by adjusting the power and flow rate, Hela cells (dyed green) stay in the acoustic action region of the bulk acoustic resonator, while leukocytes (dyed blue) and erythrocytes continue to move in the direction of fluid, leaving the microfluidic device; then, while maintaining the described power and flow rate, the input of blood containing cells is stopped and the PBS solution is input, with Hela cells remain in the acoustic action region of the resonator while the solvent solution is converted from blood to PBS; the power is then turned down and the Hela cells detach from the vortex and flow with the PBS fluid to the next microfluidic channel, i.e., the process of Hela cell capture and transfer from the blood into the PBS buffer is completed. Similarly, Hela cells can be transferred from blood or PBS buffer to other solutions, such as Trypan dye solution.

Moreover, the capture of cells by the apparatus and method of the present invention can achieve single cell separation. Since under the conditions of the method and apparatus of the present invention, i.e., when the height of the microfluidic channel is about 1.5-6 times the diameter of the target cell, more preferably about 2-4 times, the vortex channel is best suited to hold one cell in the vertical height range. In this way, the cells captured in the acoustic action region of each high-frequency bulk acoustic resonator are lined up side-by-side along the edge of the acoustic action region and are substantially free of oscillations, reducing the damage caused by contact and collision between cells. It is also easy to perform the next step of separating individual cells by changing the vortex formation conditions to make the cells leave the vortex tunnel.

It has been demonstrated that the method and apparatus of the present invention are capable of distinguishing and separating cells of different sizes, and that the resolution can be achieved by separating blood cells from other cells in the blood (e.g., circulating tumor cells such as Hela cells).

In addition, the size of the captured cells can be selected by adjusting the acoustic wave and lateral flow rates. Leukocytes and erythrocytes can also be selectively captured from blood samples, and the results are shown in Figure 7(b) and Figure 7(c). In Figure 7(b), the blood sample is stained with DAPI, so that the leukocyte WBC stain blue in the fluorescence view. Figure 7(b) shows that individual leukocytes were captured and cell activity was maintained when the power was adjusted to 13.2 mV. Figure 7(c) shows that different cells were captured at different power levels. For example, at a power of 6.6 mV, both leukocytes and erythrocytes were not captured; at a power of 13.2 mV, leukocytes were captured while neither of the erythrocytes were captured; and at a power of 20.9 mV, both leukocytes and erythrocytes were captured.

The inventors used software (COMSOL, USA) to construct a model to analyze the separation process of the aforementioned three cells. The resolution of cell separation in the simulation was much worse than that observed in the actual experiment. This may be due to factors such as cell interactions and deformation that are not represented in the simulation model.

### Example 7 Maintenance of activity of captured cells

The maintenance of cellular activity is of great importance for cell research. However, most prior art methods of cell isolation adversely affect cell activity or even kill cells. Factors that have an effect on cells in existing cell manipulation techniques include mechanical shear, Joule heat, or electron fields. When sound waves generated by UHF bulk acoustic resonators are absorbed into a fluid, the mechanical energy is converted into kinetic energy of fluid and heat. Thermal effects may lead to biological effects that may result in conformational changes, protein misfolding, entanglement and/or non-specific aggregation of proteins, etc.

The methods and apparatus of the present invention are able to maintain the full activity of the captured cells.

In the present experiments, Taipan Blue (an important stain used to selectively label dead tissues or cells) was used to inject captured cells as a probe for active cells.

The experiments and results are shown in Figure 7(a). HeLa cells were captured according to the aforementioned methods and conditions. Their activity was then detected by using Tissue Blue. It was demonstrated that the cells were exchanged between buffer and Taipan Blue solution during the viability test, which was repeated three times. At 560 seconds, the cells were still active: the cell membrane remained selectively permeable to Tissue Blue.

In addition, in the experimental setup described above, rhodamine B (a temperature-dependent fluorescent dye) was added to the solution to measure the temperature distribution around the UHF bulk acoustic resonator at different applied powers. The results, shown in Figure 8, indicate that the thermal effect of the resonator is very weak, especially under conditions of selective cell capture, such as when the applied power is less than 100 mW.

### Example 8 Isolation and capture of different cells from whole blood

In the blood environment, cells have viability and intact function, which is important for biological studies, e.g., cell metabolism, proteomics.

However, whole blood is a more challenging sample to work with than diluted blood samples. In terms of physical parameters, whole blood is more viscous and turbid, both of which can have a serious negative impact on cell manipulation. Physical fields, such as dielectric fields, magnetic fields, and hydrodynamic fields, are disordered in blood samples. In addition, high densities of cells, especially erythrocytes (10⁹/ mL), can cause strong interactions between cells, which can alter the trajectory of the specimen and affect the stability of cell motility.

The inventors demonstrated that the apparatus and method of the present invention are capable of selectively controlling and isolating target cells in whole blood.

Hela cells are dissolved in DMEM culture medium and mixed with whole blood to prepare a test sample solution (Hela cell concentration is adjusted to approximately 1×10⁵ cells/mL).

The inlets of the microfluidic system were designed in a three-flow mode, i.e., including a sample inlet in the middle and two buffer inlets on both sides of the sample inlet: the sample was injected into the microfluidic channel from the middle sample inlet; and the PBS buffer was injected into the microfluidic channel from the buffer inlets on both sides. The resulting PBS buffer added on both sides serves as a pinch sheath flow, limiting the lateral extent of the sample fluid and ensuring that all samples pass over the UHF bulk acoustic resonator located in the center of the flow channel. Passive focusing of the injected sample by means of three-flow mode allows sample flow through the acoustic action region of the UHF bulk acoustic resonator in a better way.

The microfluidic device of the present invention is optimized for better separation of target cells and increased separation efficiency. For example, the setting of the acoustic wave action region of the high-frequency bulk acoustic wave resonator is adjusted to separate the capture point of target cells from the release point of non-target cells.

The bulk acoustic wave action region of the UHF wave resonator of this embodiment is set up in such a way that when treating cells containing different sizes, the location of the captured target cells (or called the capture point) is different from the non-target cells leaving the action region (or called the release point). For example, if the bulk acoustic wave region of the UHF bulk acoustic wave resonator is pentagonal, it is set up so that one of the corners is symmetrically placed along the midline of the flow channel in the direction of the channel fluid inflow: the location of the non-target cells leaving the bulk acoustic wave region, or the release point, is at the ends of the downstream edge along the fluid direction, while the location of the target cells being captured, or the capture point, is in the middle of the downstream edge along the fluid direction. The capture point and the release point do not overlap with each other. As a result, the non-target cells have the least negative effect on the stabilizing of the captured cells.

The results are shown in Figure 9, where Hela cells in whole blood are selectively captured.

Figures 9(c) and 9(d) show the photographs of Hela cells are captured while blood cells pass through the bulk acoustic wave region under the two microfluidic channel setups, and Figures 9(a) and 9(b) are the analysis and schematic diagrams of Figures 9(c) and 9(d), respectively. In this embodiment, the bulk acoustic wave effecting region of the UHF bulk acoustic wave resonator is pentagonal. In Figure 9(c), the bulk acoustic wave effecting region is set to have a protruding point (one corner of the pentagon) downstream of the liquid inflow direction: the positions where the blood cells leave the bulk acoustic wave effecting region is near said corner of the pentagon; and the location of the captured Hela cells, i.e., the capture point, is also near the same corner. In such case, the blood cells have an effect on the stability of the captured Hela cells. In Figure 9(d) the bulk acoustic wave action region is adjusted to have one of the corners of the pentagon placed along the midline of the flow channel and opposite to the direction of fluid inflow into the channel; the releasing points of the blood cells, are located at the two end points of the downstream side line of the pentagon, while the capture point of the Hela cell is in the middle of the downstream edge along the fluid direction: the capture point and the release point do not overlap with each other. In such case, the blood cells have the least negative effect on the stability of the captured Hela cells. Figure 9(e) shows the distribution of blood cells upstream and downstream of the region of bulk acoustic wave action (regions separated by the lines in the photos in Figures 9(c) and 9(d)). The curves indicate that the blood cells are released evenly at two symmetric release points without passing the capture point. Figure 9(f-1) shows that the three-flow mode setting: the injected sample solution in the middle is passively focused by the two PBS solution at both sides, allowing better sample flow through the acoustic effecting region of the high-frequency bulk acoustic resonator. Figure 9(f-2) shows the flow of blood cells when the power is turned on and bulk acoustic waves are generated within the microfluidic channel. Figures 9(f-3)-9(f-5) show CTCs being selectively captured: the composite stacked image sequence demonstrates the captured CTCs motion trajectory. Figure 9(f-6) shows the results of selective CTC capture: the merged images show that the device provided by the present invention allowing CTCs' selectively entry and stably retained in the vortex tunnel, while allowing the releasing of blood cells.

In a better method of the present invention, in order to reduce the negative effects caused by cell-cell interactions, the bulk acoustic wave action region of a UHF bulk acoustic wave resonator in the microfluidic device is adjusted to distinguish between the capture point of the target cells and the release point of non-target cells. In the case of isolation of incorporated Hela cells from diluted blood samples, the negative effects triggered by the interaction between captured cells (Hela cells) and released cells (blood cells) are acceptable due to the relatively low density of blood cells. However, in the case of separation from undiluted whole blood, the high viscosity of the blood limits the velocity gradient of the fluid and the extremely high cell density triggers collisions and friction between cells. Captured Hela cells may deviate from their original trajectory or capture point in response to the interaction of other cells. By adjusting the region of action of the bulk acoustic wave of the UHF bulk acoustic wave resonator of the microfluidic device to separate the capture point from the release point, and in particular by placing the release point before the capture point (upstream), non-target cells are carried away by the laminar flow without passing the capture point, and only the target cells can reach the capture location.

### Example 9 Capture of CTCs in Patient Blood Samples

Circulating tumor cells, or CTCs, are tumor cells found in the circulation of a patient with a tumor. The term generally does not include blood tumor cells. In a patient's circulation, CTC levels are extremely low and can be less than 1 CTC per milliliter of blood. Circulating tumor cells are clinically important for the diagnosis of cancer. Detecting CTCs in the blood or isolating CTCs from the blood presents significant challenges due to the scarcity of CTCs and the presence of a large number of cells with similar physical properties in the blood.

The present application demonstrates that the method and apparatus of the present invention are capable of isolating and capturing CTCs from the blood of patients with hepatocellular carcinoma.

A 1 ml blood sample from three patients with stage IV liver cancer was injected from the sample inlet into a microfluidic channel. PBS buffer and staining solution containing Calcein-AM and anti-EpCAM antibody labeled with red fluorescence were injected into the microfluidic channel from the inlet on each side, respectively. The central sample flow rate was controlled to range from about 1 µL/min, or about 0.67 mm/s, while the PBS flow rate was about 5 µL/min.

The results are shown in Figure 10. Figure 10 (a-1) shows the distribution of fluid in the microfluidic channel without turning on the power: the PBS stream enters the flow channel from the top left channel, the blood sample enters from the top right channel, and the staining solution is fed into the bottom right channel; under the entrainment effect of the PBS stream and the staining solution, the blood sample flows through the bulk acoustic wave generation region of the pentagonal high-frequency bulk acoustic wave resonator set near the right side of the flow channel. Figure 10(a-2) shows that when power is applied, the power is adjusted to 30 mW, the resonator generates vortex in the microfluidic channel, blood cells pass through the bulk acoustic wave generation region, and target cells are captured; then the blood sample and PBS buffer inlet are closed and the dye solution occupies the bulk acoustic wave generation region to stain the cells. After staining, the dye inlet is closed and PBS buffer is injected to wash the remaining blood cells and dye. Figure 10 (a3-5) shows CTCs captured in the acoustic generation region and blood cells were not captured (passing through the acoustic signal generation region). Figure 10(b) shows staining of a captured single target cell. The cell was stained green by Calcein-AM, showing that the captured cell remains active, while the red fluorescence of the anti-EpCAM marker represents the presence of the cancer marker EpCAM in the captured cell.

The 3T3 cell line (3T3 is a cell line with low expression of EpCAM) was used as a negative control in the experiment. The experimental procedure was the same as previously described in Figure 10(a), and the results are shown in Figure 10(c). The results in Figure 10(c) show that both CTC and 3T3 were stained with Calcein-AM (green), CTC was stained with anti-EpCAM antibody, while 3T3 was not stained red because EpCAM was hardly expressed on it. The results proved that the captured single cells or cell clusters were cancer cells.

It was demonstrated that the method of the present invention can capture CTCs in patient whole blood samples and perform in situ staining. Compared to staining in centrifuge tubes by centrifugation and/or magnetic beads, for example, the method of in situ staining analysis provided by the present invention offers significant advantages, including the elimination of the need to repeat the steps of centrifugation and washing and changing dyes or solutions.

### Embodiment 10 Controlled release of cells

The inventors of the present application also unexpectedly found that in the method and apparatus of the present invention, within a certain range, the number of cells captured in the vortex tunnels generated by the UHF bulk acoustic wave resonator is linearly related to the power applied to the UHF bulk acoustic wave resonator, thereby providing controlled release of cells, particularly a few or individual cells.

Hela cell samples and markers and microfluidic channels are prepared according to the method of Example 3. The sample is injected from the sample inlet into the microfluidic channel and passed through the vortex tunnel generated by UHF bulk acoustic wave resonator.

As shown in Figure 11(a), the number of cells captured in the vortex tunneling generated by the UHF bulk acoustic wave resonator is influenced by the flow rate of the cells and the power of bulk acoustic wave.

The inventors unexpectedly found a linear relationship between the number of captured cells (N), the applied power (P), and the lateral fluid velocity (V), within a certain range. The inventors found experimentally that N=0.4^{∗}P when V=0.33 mm/s; N=0.28^{∗}P when V=0.67 mm/s; N=0.18^{∗}P when V=1.34 mm/s; and N=0.15^{∗}P when V=2 mm/s; wherein the coefficient k ≈ 0.47^{∗}exp(-V/0.83)+0.14. This equation is applicable when intercellular interactions are less and weaker, and when no cell-to-cell adhesion and cell clusters occurs.

The inventors found through experiments that the preferred conditions are: P<100 mW at V<1 mm/s; P<300 mW at 1 mm/s<V<3 mm/s. For ensuring the accuracy of selectively isolating Hela cells, the lateral flow velocity is most preferably about 0.5-1 mm/s and the applied power is preferably at 5-20 mW, as so, the number of cells captured by a single resonator is between 1 and 6. If a larger number of cells is to be isolated, more resonators can be used in the device.

The following table shows the results of the number of captures.

| Flow velocity(V)/power(P) | 0.33 mm/s | 0.67 mm/s | 1.34 mm/s | 2 mm/s |
|---|---|---|---|---|
| 1mW | 1 | 0 | 0 | 0 |
| 5mW | 3 | 1 | - | - |
| 10mW | 4 | 3 | 1 | - |
| 15mW | 8 | 6 | 2 | 1 |
| 40mW | 15 | 10 | 9 | 4 |
| 80mW | - | - | 14 | 11 |
| 160mW | - | - | - | 26 |

Thereby, the present application also provides methods of controlling cell capture and release, in which the number of captured cells as well as the number of released cells can be controlled; by controlling the cells being released one by one, single cell is obtained. As an example, in Figure 8(b), by reduce the power gradually, the captured cells can be released in a sequential manner. For example, for cells with diameters of 15-20 µm, the power to capture multiple cells at a lateral flow rate of 0.67 min/s is about 35 mW. A power of less than 100 mW is preferred; higher power may lead to the appearance of intercellular adhesions, which is not desired to the operation of single cells. Cell capture is performed at an input power of 30-35 mW, and when the target number of cells is reached, a gradient of power reduction is started, e.g., 5-20% decrease of the power, e.g., about 10%. The smaller the gradient the more precise the manipulation, but the longer time it took for the cells being captured, the more likely intercellular adhesions will occur.

As shown in Figure 8(b), with a power of -33 Mw is applied, two cells are captured; with a power of ∼13 mW, one single cell is released; and a complete release is obtained at power of -0.1 mW; when three cells are captured, a first cell is released at power of ∼20 mW, a second cell is released at power of -16.5 mW, and a third cell is released at power of -0.1 mW; when four cells are captured, the first cell is released at power of about 21 mW, the second cell is released at power of about 17 mW, the third cell is released at power of about 6.6 mW, and the fourth cell is released at power of about 0.1 mW.

It can be seen, in the system and method of the present invention, the number of cells captured in the vortex can be adjusted by modulating the applied power. The captured cells are aligned along the boundary of the bulk acoustic wave action region of the UHF bulk acoustic wave resonator, and under the action of the lateral fluid, the cells have a tendency to move downstream along the device boundary, so that the first cells to be shed under changing capture conditions are the cells that are at the most downstream boundary. By gradually reducing the power, the ability of the vortex to capture cells gradually decreases, and when the power is reduced to the point where the vortice channel cannot hold all of the captured cells, the cells closest to the downstream will disengage from the vortex, and the other capturing cells will move in sequence to fill the space left vacant by the released cells. Therefore, a controlled release of the captured cells can be achieved by reducing the applied power. It is worth emphasizing that this release can be achieved at the single cell level.

### Example 11 Assembly of cells with gel microspheres

Based on the finding that the relationship between the increase in applied power and the number of captured cells or other flexible particles is linear in the system and method of the present invention, the number of captured cells and/or other particles in the vortex can be adjusted by modulating the applied power, which in turn also allows for a controlled release of captured cells or other particles by reducing the applied power in a gradient.

In the present experiments, methods for capturing individual cells and assembling them with individual gel microspheres for further analysis such as sequencing of single cells were tested.

The method of this embodiment includes the following steps.
(1) injecting from the sample inlet a sample of cells (cell content diluted to a level suitable for capturing a single to about ten cells in the bulk acoustic wave action region of the UHF bulk acoustic wave resonator), by adjusting the bulk acoustic wave power and flow rate of the UHF bulk acoustic wave resonator such that a single cell is captured in the bulk acoustic wave action region of the UHF bulk acoustic wave resonator;
(2) injecting gel microspheres from the sample inlet and adjusting the bulk acoustic wave power and flow rate of the UHF bulk acoustic wave resonator such that individual gel microspheres enter the bulk acoustic wave action region of the UHF bulk acoustic wave resonator and are captured under conditions that maintain the individual cells captured in step (1) without being released, so that said gel microspheres are paired one-to-one with said captured cells;
(3) controlling the vortex intensity by gradually reducing the power such that the paired gel microspheres-cells are released.

In single-cell studies such as single-cell sequencing, where gel microspheres (which containing primers, lysate, etc.) need to be assembled with single cells, current assembly methods are based on a Poisson distribution in a random manner, requiring a large number of gel microspheres and a very low concentration of cells to ensure that each cell can be wrapped in the same drop with a single gel microsphere to complete the subsequent reaction. Such a technique results in a large amount of wasted gel microspheres and, more importantly, the assembly process is random and uncontrollable, with no pre-screening of the desired cells and no guarantee that a given cell will be wrapped in a gel microsphere. Using the single cell capture capability of the acoustic fluid vortex, the inventors capture single cells in the acoustic fluid system provided by the present invention and then pass through a single gel microsphere, in which the cells and microspheres are able to complete pre-assembly in the acoustic fluid tunnel. This makes it possible to identify the single cell to be studied and also ensures that this cell can be assembled with the gel microspheres.

Hela cell samples and markers as well as microfluidic channels were prepared according to the method of Example 3. The gel microspheres used for the experiments were from 10X genomics' Barcode Gel bead, which is a standard microsphere used for single cell sequencing. The experiments and results are shown in Figure 12. The applied power is 26.3 mW and the flow rate is 1 µL/min. The inlet of the microfluidic system can be used for inputting the buffer (PBS), HeLa cell solution or Barcode gel bead sample, respectively. The parameters of the system are adjusted so that both individual cells and individual barcode gel bead can enter the bulk acoustic wave region of the UHF bulk acoustic wave resonator and be captured. The procedure for this method consists of: first adding buffer; after the buffer has been excluded of air bubbles in the flow channel, the cell flow channel is opened and Hela cells are input. After a Hela cell is captured in the bulk acoustic region of the UHF bulk acoustic resonator (as shown in Figure 12a), the cell flow channel is closed and the Barcode gel beads flow channel is opened and a single Barcode gel bead is input (as shown in Figure 12b). A single cell with a single Barcode gel bead is captured in the same UHF bulk acoustic region of the bulk acoustic resonator (as shown in Figure 12c). The buffer flow channel is then turned on and the applied power is turned off to achieve the release of the paired cells and gel microspheres (as shown in Figure 12d).

The above description is only an embodiment of the present invention and is not intended to limit the present invention. Any modification, equivalent replacement, improvement, etc. made within the spirit and principles of the present invention shall be included in the scope of protection of the present invention.

## Claims

1. A method for isolating a target cell or vesicle, comprising.
(1) allowing a solution containing cells or vesicles to flow through a microfluidic device, said device comprising :
a fluid channel having an inlet and an outlet;
one or more ultra-high frequency bulk acoustic wave resonators provided on a wall of said fluid channel, said ultra-high frequency bulk acoustic wave resonators being capable of generating bulk acoustic waves in said fluid channel with a frequency of about 0.5-50 GHz and transmitted to the opposite side of said fluid channel;
(2) said UHF resonator emits a bulk acoustic wave transmitted to said wall on the opposite side of said fluid channel, creating a vortex in the solution in the area affected by the bulk acoustic wave;
(3) causing cells or vesicles to remain in the area affected by the bulk acoustic wave by adjusting the power of the bulk acoustic waves and/or adjusting the velocity of flow of said solution through the area affected by the bulk acoustic wave.

2. The method of claim 1, wherein the power of the bulk acoustic wave generated by said UHF resonator is adjusted to be about 0.5-800 mW, preferably 0.5-500 mW, more preferably 0.5-350 mW of the bulk acoustic wave.

3. The method of claim 1, wherein the velocity of flow of said solution through the bulk acoustic wave region is regulated to be about 0.1-10 mm/s, preferably about 0.3-5 mm/s, more preferably about 0.5-2.5 mm/s.

4. The method of claim 1, wherein the velocity of flow of said solution through the bulk acoustic wave region is regulated to be about 0.1-100 µL/min, preferably about 0.1-50 µL/min, more preferably about 0.5-20 µL/min.

5. The method of claim 1, wherein said cell or vesicle has a diameter of about 0.8-30 um, for example about 1-25 um, furthermore for example about 5-20 um.

6. The method of claim 1, wherein said fluidic channel of said microfluidic device has a height of about 25-200 µm, preferably about 25-100 µm, more preferably about 30-80 µm, such as about 40-60 µm.

7. The method of claim 1, wherein said UHF bulk acoustic wave resonator has a bulk acoustic wave generating area of about 500-200000 µm², preferably about 5000-50000 µm², most preferably about 10000-25000 µm².

8. The method of claim 1, which is for separating a single cell or several cells or a cluster of cells.

9. The method of claim 1, which comprises controlling the cells or vesicles and/or the number thereof that remain in said area affected by the bulk acoustic wave, for example by one of the following or any combination thereof:
(a) adjusting the power of the bulk acoustic wave;
(b) adjusting the time for which the bulk acoustic waves are generated;
(c) adjusting the velocity of flow of said solution through the region of the bulk acoustic wave.

10. The method of claim 1, wherein further comprising causing the remaining cell or vesicle to leave the remaining position, for example by one way as shown below or any combination thereof:
(a) stopping the bulk acoustic wave;
(b) decreasing the power of said bulk acoustic wave;
(c) increasing the velocity of the solution flow through the region of the bulk acoustic wave.

11. The method of claim 1, which is for separating a cell or vesicle to be isolated from a solution containing other cells or vesicles, preferably wherein said cell or vesicle to be isolated has a ratio of less than 1:10⁴ to other cells or vesicles, preferably a ratio of less than 1:10⁵, more preferably a ratio of less than 1:10⁶.

12. The method of claim 11, wherein the bulk acoustic wave effecting region of said ultra-high frequency bulk acoustic wave resonator of said apparatus is set in such a way that the location of the target cell or vesicle being captured is different from the location of other cells leaving the bulk acoustic wave action region, preferably, the location of other cells or vesicles leaving the bulk acoustic wave action region is set upstream of the location of the target cell or vesicle being captured.

13. The method of claim 1, wherein said cell is a eukaryotic cell, preferably a mammalian cell, more preferably a human cell.

14. The method of claim 1, wherein said cell is a blood cell, a stem cell, a bulkcell or a tumor cell, for example a circulating tumor cell (CTC).

15. The method of any one of claims 1-14, which further comprises causing a single cell or vesicle to remain in a bulk acoustic wave affected region, and then assembling said single cell or vesicle with a single microcapsule.

16. The method of claim 15, wherein the method comprises the steps of:
(a) allowing a single cell or vesicles to remain in the region of bulk acoustic wave influence of the UHF bulk acoustic wave resonator by adjusting parameters of the UHF bulk acoustic wave resonator, such as by adjusting the bulk acoustic wave power and flow rate;
(b) injecting gel microspheres from the sample inlet, adjusting the parameters of the UHF bulk acoustic wave resonator, for example by adjusting the bulk acoustic wave power and flow rate, such that a single or several microcapsules also remain in the bulk acoustic wave action region of the UHF bulk acoustic wave resonator, while maintaining the cells or vesicles remaining in step (a) and preventing them from being released;
(c) said microcapsules assemble with said remaining cells or vesicles,
preferably, wherein further comprising (d) allowing the assembled microcapsule-cell/vesicle to be released by reducing the bulk acoustic wave power or stopping the bulk acoustic wave.

17. A microfluidic device for isolating a target cell or vesicle, comprising
a fluid channel having an inlet and an outlet;
one or more ultra-high frequency bulk acoustic wave resonators provided on a wall of said fluid channel, said ultra-high frequency bulk acoustic wave resonators being capable of generating bulk acoustic waves in said fluid channel with a frequency of about 0.5-50 GHz and transmitted to the opposite side of said fluid channel;
a power adjusting device which adjusts the power of said bulk acoustic waves generated by said UHF resonator;
a flow rate adjusting device which adjusts the velocity of said solution flowing through the region of the bulk acoustic wave,
said UHF resonator emitting bulk acoustic waves transmitted to the opposite wall of said fluid channel, causing the solution flowing through the bulk acoustic wave region to generate vortices, adjusting the power of the bulk acoustic waves by said adjusting device and/or adjusting the velocity of said solution flowing through the bulk acoustic wave region by said flow rate adjusting device, causing the cells or vesicles to stay in the bulk acoustic wave region.

18. The microfluidic device of claim 17, wherein said power adjusting device outputs power of about 0.5-800 mW, preferably 0.5-500 mW, more preferably 0.5-350 mW.

19. The microfluidic device of claim 17, wherein said flow rate adjusting device adjusts the velocity of said solution flow through the bulk acoustic region to about 0.1-10 mm/s, preferably about 0.3-5 mm/s, more preferably about 0.5-2.5 mm/s.

20. The microfluidic device of claim 17, wherein said flow rate adjustment device adjusts the rate of flow of said solution through the bulk acoustic region to about 0.1-100 µL/min, preferably about 0.1-50 µL/min, more preferably about 0.5-20 µL/min.

21. The microfluidic device of claim 17, wherein said fluid channel of said microfluidic device has a height of about 25-200µm, preferably about 25-100µm, more preferably about 30-80µm, for example about 40-60µm.

22. The microfluidic device of claim 17, wherein said ultra-high frequency bulk acoustic wave resonator has a bulk acoustic wave generating area of about 500-200000 µm², preferably about 5000-50000 µm², most preferably about 10000-25000 µm².

23. The microfluidic device of claim 17, wherein said microfluidic device is set in such a way that the location of the target cell or vesicle being captured is different from the location of other cells leaving the bulk acoustic wave action region.

24. The microfluidic device of claim 23, wherein the location of other cells or vesicles leaving the bulk acoustic wave action region is set upstream of the location of the target cell or vesicle being captured.

25. The microfluidic device of claim 17, wherein said UHF bulk acoustic wave resonator is a thin film bulk acoustic wave resonator or a solid state assembled resonator, such as a thickness stretching vibration mode acoustic wave resonator.
